# FASCICULE DE BREVET EUROPEEN

(11) **EP 1 615 560 B1**
(45) Date de publication et mention de la délivrance du brevet: **30.05.2007**
(21) Numéro de dépôt: 04742562.4
(22) Date de dépôt: 22.04.2004
(51) Int. Cl.: A61B 6/00

(54) **FANTOME POUR LE CONTROLE QUALITE D'UN SYSTEME DE SIMULATION VIRTUELLE D'UN TRAITEMENT DE RADIOTHERAPIE**
PHANTOM FÜR DIE QUALITÄTSKONTROLLE EINES SYSTEMS ZUR VIRTUELLEN SIMULATION EINER RADIOTHERAPEUTISCHEN BEHANDLUNG
PHANTOM WHICH IS INTENDED FOR THE QUALITY CONTROL OF A VIRTUAL RADIOTHERAPY TREATMENT SIMULATION SYSTEM

(30) Priorité: 23.04.2003 FR 0304987
(43) Date de publication de la demande: 18.01.2006
(73) Titulaire: ASSISTANCE PUBLIQUE - HOPITAUX DE PARIS, 75001 Paris (FR)
(72) Inventeur: FOULQUIER, Jean-Noël, F-94130 NOGENT-SUR-MARNE (FR); EL-BALAA, Hanna, F-94407 VITRY-SUR-SEINE (FR); LEFKOPOULOS, Dimitri, F-77140 NEMOURS (FR)
(74) Mandataire: Neyret, Daniel Jean Marie
(86) Numéro de dépôt international: PCT/FR2004/000987
(87) Numéro de publication internationale: WO 2004/096047

(56) Documents cités:
- DE-A- 19 819 928
- US-A- 4 055 771
- US-A- 5 416 816
- US-B1- 6 409 515

## Description

La présente invention concerne le domaine de la radiothérapie. Plus précisément, elle concerne un dispositif du type connu sous le nom de "fantôme" utilisé pour la préparation de l'appareillage lors des opérations de simulation virtuelle préparant l'exécution d'un traitement de radiothérapie au moyen d'un scanner ou analogue.

Le traitement des tumeurs par radiothérapie est aujourd'hui possible en utilisant des appareils qui comprennent un dispositif d'imagerie médicale, tel qu'un scanner, couplé à un dispositif d'émission du rayonnement utilisé pour le traitement du patient. La préparation de ces appareils, avant la réalisation effective de l'irradiation du patient comporte une étape dite de "simulation virtuelle".

Ce terme se réfère à un ensemble de logiciels qui permet à l'utilisateur de définir ou de calculer ce que l'on appelle l'"isocentre de traitement", c'est-à-dire la zone ponctuelle où doit converger le rayonnement devant détruire la tumeur, puis de simuler le traitement à effectuer à l'aide de clichés radiologiques reconstruits. Deux composantes logicielles sont utilisées lors de la phase de simulation virtuelle:
- des logiciels qui définissent les contours d'une part de la tumeur à traiter et d'autre part des organes qu'il importe de ne pas atteindre lors de l'émission du rayonnement ;
- et des logiciels qui permettent de placer les faisceaux grâce à la vision des clichés radiologiques reconstruits et de positionner les caches ou les lames du collimateur.

Le dispositif assurant la simulation virtuelle propose tous les mouvements d'un accélérateur de particules à l'aide des logiciels paramétrés. Mais classiquement, la pertinence des données fournies par ces logiciels ne peut être contrôlée que lors d'une simulation effectuée en présence du patient, ce qui est contraignant pour ce dernier. Il est donc souhaitable de disposer d'un outil qui permettrait de réaliser un contrôle de la pertinence du fonctionnement des logiciels de simulation virtuelle en dehors de la présence du patient.

Il est connu d'utiliser, pour l'étalonnage des scanners, des dispositifs appelés "fantômes". Ils sont constitués par un volume de dimensions connues d'un matériau (eau, polystyrène de diverses densités, plexiglas) se comportant essentiellement de la même façon que le tissu humain qui est concerné par l'examen, du point de vue de l'absorption et de la diffusion du rayonnement utilisé. Ces fantômes connus ne sont pas adaptés à la réalisation d'une simulation virtuelle telle qu'on vient de la définir.

Il a été proposé dans le document "A quality assurance phantom for digitally reconstructed radiograph (DRRs) Med Phys 1994 . 21, 902", d'utiliser un fantôme constitué d'un cadre de polystyrène de 15 cm de côté, comportant quatre faces test. Il permet de réaliser une évaluation de la résolution spatiale de l'appareil. Sur la face principale sont gravées des formes géométriques qui permettent de mesurer la fonction de transfert de modulation, la finesse du contraste, la linéarité spatiale des clichés radiologiques reconstruits et la qualité de l'algorithme de reconstruction des clichés radiologiques reconstruits pour un faisceau divergent. Mais ce fantôme ne permet pas de réaliser toutes les opérations nécessaires pour vérifier la qualité de la simulation virtuelle. Il est donc toujours nécessaire, pour contrôler la qualité d'une simulation virtuelle dans son ensemble, de réaliser plusieurs analyses successives d'objets tests différents, ce qui demande une disponibilité importante du scanner et de la console de simulation virtuelle.

Le but de l'invention est de proposer un fantôme permettant de tester l'ensemble des fonctions de simulation virtuelle d'un système de radiothérapie utilisant un dispositif d'imagerie tel qu'un scanner, au cours d'un nombre minimal d'opérations.

A cet effet, l'invention a pour objet un fantôme pour le contrôle qualité d'un système de simulation virtuelle d'un traitement de radiothérapie comportant un dispositif d'imagerie médicale, caractérisé en ce qu'il comporte:
- un caisson support,
- un noyau disposé dans ledit caisson support et constitué par une pluralité d'éléments de formes, dimensions et densités différentes, lesdites densités simulant les densités de divers organes et milieux du corps humain, deux de ces éléments étant constitués par deux troncs de pyramides de densités différentes emboîtés l'un dans l'autre, l'un d'entre eux au moins ne présentant pas une totale symétrie par rapport à son axe longitudinal,
- des billes en un matériau non radiotransparent disposées dans ledit noyau,
- au moins deux faces latérales amovibles opposées l'une à l'autre renfermant des fils métalliques définissant des figures géométriques.

De préférence, le fantôme est de forme générale cubique.

De préférence, l'une des billes est placée au centre du noyau.

De préférence, le fantôme comporte six faces latérales amovibles renfermant des fils métalliques définissant des figures géométriques.

Comme on l'aura compris, le fantôme selon l'invention se compose en premier lieu d'un caisson support, de préférence de forme cubique. Sur ce caisson support sont montées au moins deux plaques sur lesquelles est matérialisée une figure géométrique à l'aide de fils métalliques qui y sont noyés, placées à l'opposé l'une de l'autre sur le caisson support. Ces deux plaques servent à vérifier que la divergence de l'image reconstruite est correcte. Avantageusement, toutes les faces du caisson support comportent de telles plaques, de sorte qu'il est possible de réaliser un maximum de tests de divergence au cours d'une seule opération. Le caisson est garni intérieurement par des volumes présentant des formes géométriques remarquables ainsi que des densités différentes, permettant de simuler les densités de divers organes (tels que le sein, les muscles, les os, les poumons remplis d'air). L'un de ces volumes, en particulier, est constitué par des troncs de pyramide emboîtés. A l'intérieur de ces volumes sont placées un certain nombre de billes métalliques, de préférence en acier, à des positions définies. De préférence, l'une de ces billes est placée au centre du fantôme. Ces billes constituent des repères d'isocentres de traitement. La comparaison entre l'image des volumes garnissant le caisson et la réalité permet de vérifier le bon étalonnage des logiciels de simulation virtuelle.

L'invention sera mieux comprise à la lecture de la description qui suit, donnée en référence aux figures suivantes :
- la figure 1, qui représente en perspective un exemple de caisson support pour un fantôme selon l'invention et les faces latérales qui lui sont associées
- la figure 2, qui représente en perspective un exemple de noyau destiné à être inséré dans ledit caisson support ;

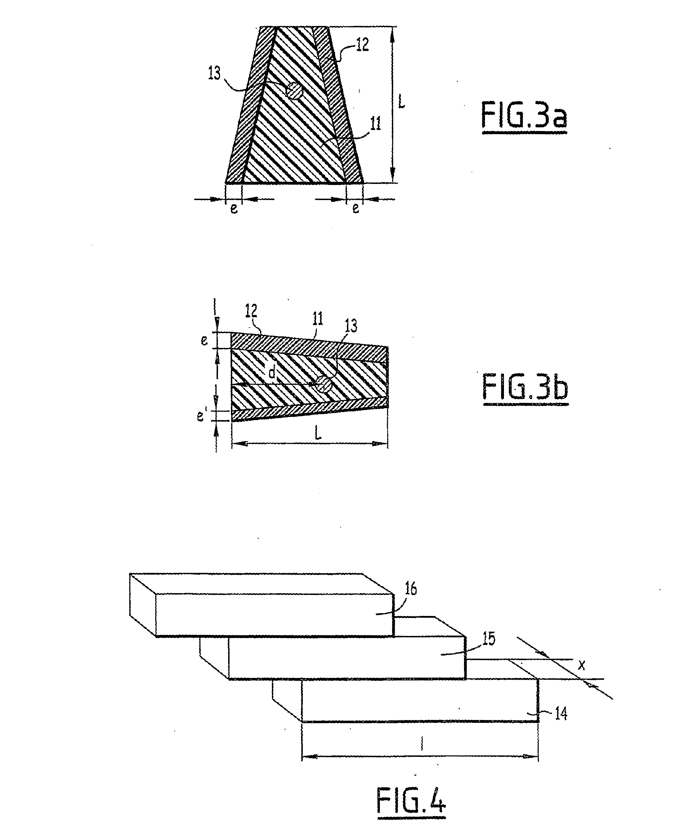

- la figure 3, qui représente vu en coupe selon III-III (figure 3a) et en coupe selon IV-IV (figure 3b) une partie dudit noyau ;
- la figure 4, qui représente en perspective une autre partie dudit noyau.

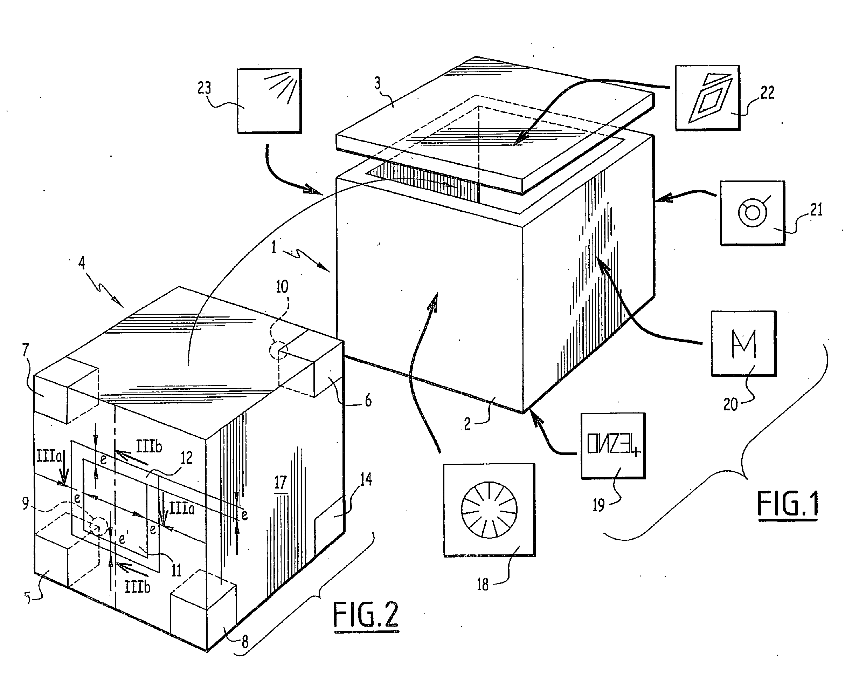

Le caisson support 1 représenté sur la figure 1 est constitué par l'assemblage de deux éléments qui, dans l'exemple représenté, constituent un cube de 19 cm de côté. Le premier élément 2 est une boîte ouverte sur sa face supérieure, dont la base est un carré de 19 cm de côté et dont les faces latérales ont une hauteur de 18 cm. Le deuxième élément 3 est une plaque carrée de 19 cm de côté et de 1 cm d'épaisseur qui est disposée à la partie supérieure du caisson 1 de manière à former un couvercle pour le premier élément 2. Ces deux éléments 2, 3 sont en un matériau tel que le polystyrène choc (de densité 1,05), mais pourraient aussi être en polyméthylméthacrylate (PMMA) par exemple. Ces deux éléments 2, 3 sont solidarisés au moyen de vis en un matériau tel que le nylon, de longueur 2 cm.

Le caisson support 2 est destiné à renfermer un noyau 4 dont un exemple est représenté sur la figure 2.

Ce noyau 4 est constitué par un assemblage cubique de 17 cm de côté, composé d'éléments dont certains possèdent des propriétés géométriques remarquables, et présentant des densités diverses représentatives des densités des divers organes et milieux du corps humain que les rayonnements du dispositif de radiothérapie sont susceptibles de traverser. Parmi ces éléments se trouvent quatre cubes 5, 6, 7, 8 de 3,5 cm de côté qui occupent chacun un sommet du noyau 4. Deux de ces cubes 5, 6 sont placés sur deux sommets diagonalement opposés. Ces cubes 5, 6, 7, 8 sont tous de densités différentes. Par exemple, le cube 5 a une densité de 0,991 simulant celle du sein, le cube 6 a une densité de 1,609 simulant celle de l'os, le cube 7 a une densité de 1,062 simulant celle du muscle et le cube 8 a une densité de 0,465 simulant celle du poumon exhalé Sur les sommets des cubes 5, 6 situés aux points ayant les coordonnées (5;5;5) et (-5;-5,-5) dans le repère ayant pour origine le centre du noyau 4 sont placées des billes en un matériau non radiotransparent tel que l'acier 9, 10 destinées à simuler des isocentres de traitement. L'acier est choisi de préférence à d'autres matériaux car il est bien visible sur les images reconstruites et ne provoque pas trop d'artefacts sur cette image.

D'autres de ces éléments sont constitués par un élément 11 en forme de tronc de pyramide de longueur "L" = 13,5 cm, dont la grande base est un carré de 5,5 cm de côté, et par un élément 12 qui enveloppe l'élément 11 et possède une forme extérieure non totalement symétrique par rapport à son axe longitudinal. Il entoure l'élément 11 sur une épaisseur "e'" = 1 cm selon trois des côtés de sa grande base et sur une épaisseur "e" = 0,5 cm selon le quatrième côté. Ces deux éléments 11, 12 ont des densités différentes. L'ensemble formé par ces deux éléments 11, 12 est destiné à former la partie centrale du noyau 4. Il a une longueur "L" de 13,5 cm, donc inférieure à la longueur d'une arête du noyau 4 qu'il ne traverse donc pas de part en part. De préférence, au sein de l'élément 11, à une distance "d" de la grande base égale à 8,5 cm, se trouve une bille d'acier 13 simulant un isocentre de traitement. la distance "d" est choisie pour que la bille 13 se trouve exactement au centre du cube formé par le noyau 4 assemblé.

Dans la partie du noyau 4 située en retrait des éléments en tronc de pyramide 11, 12, on intègre également trois éléments 14, 15, 16 parallélépipédiques de longueur "l" = 6 cm, et de largeur et épaisseur égales à 2 cm. Ces éléments sont disposés en étant superposés et décalés longitudinalement les uns par rapport aux autres. Là encore, ils ont chacun des densités différentes simulant divers constituants du corps humain.

Le restant 17 du noyau 4 cubique, dans lequel sont placés les différents éléments 5, 6, 7, 8, 11, 12, 14, 15, 16, est constitué par un corps en polystyrène.

Enfin, le caisson 1 est revêtu sur ses six faces par des plaques 18, 19, 20, 21, 22, 23 carrées de 20 cm de côté et de 0,5 cm d'épaisseur qui lui sont fixées de manière amovible par des vis de 1 cm de longueur en un matériau radiotransparent tel que du nylon. Ces plaques 18-23 sont en plexiglas, et elles comportent, noyés dans leur masse, des fils en un métal tel que du cuivre qui dessinent des figures géométriques telles que celles représentées sur la figure 1. Chacune de ces figures peut être dédiée à la vérification d'une ou de plusieurs fonctions particulières du logiciel, au vu de la manière dont le logiciel a restitué leur forme. Le fait d'avoir six telles plaques amovibles 18-23 est avantageux en ce qu'il permet de tester un maximum de onctions au cours d'un seul essai. Il demeurerait dans l'esprit de l'invention de prévoir un nombre de plaques amovibles moins élevé. Toutefois, un minimum de deux plaques 18-23 disposées sur deux faces opposées du noyau 4 est nécessaire pour vérifier que la divergence de l'image reconstruite est correcte. A cet effet, des motifs dessinant des cercles comme représenté sur les plaques 18, 21 de la figure 1 sont particulièrement indiqués.

Le fantôme qui vient d'être décrit et représenté n'est qu'un exemple; En particulier, il demeurerait dans l'esprit de l'invention de lui conférer une forme autre que cubique. Le cube présente l'avantage d'une grande facilité de manipulation et d'une interchangeabilité des plaques 18-23 constituant ses faces extérieures. Les formes et les dimensions des éléments formant le noyau 4 peuvent être différentes de celles qui ont été décrites. Toutefois, la présence d'éléments emboîtés en troncs de pyramide 11, 12 de densités différentes est indispensable.

Cela permet de vérifier:
- la capacité du logiciel à effectuer avec précision des contourages automatiques d'organes de différentes densités ;
- la précision des marges de sécurité qui peuvent être affectées à un organe qui va bouger en cours de traitement ; le fait que l'élément 12 soit dissymétrique est voulu pour vérifier une expansion d'organe non symétrique ; connaissant les épaisseurs des éléments 11 et 12 il est possible de comparer la mesure de ces éléments au calcul de la marge fait par le logiciel qui doit correspondre à l'épaisseur du matériau de l'élément extérieur 12 ;
- la capacité du logiciel à interpoler des contours différents, on peut ainsi estimer la précision de la reconstruction du volume.

## Revendications

1. Fantôme pour le contrôle qualité d'un système de simulation virtuelle d'un traitement de radiothérapie comportant un dispositif d'imagerie médicale, **caractérisé en ce qu'**il comporte :
- un caisson support (1),
- un noyau (4) disposé dans ledit caisson support (1) et constitué par une pluralité d'éléments (5, 6, 7, 8, 11, 12, 14, 15, 16, 17) de formes, dimensions et densités différentes, lesdites densités simulant les densités de divers organes et milieux du corps humain, deux de ces éléments (11, 12) étant constitués par deux troncs de pyramides de densités différentes emboîtés l'un dans l'autre, l'un d'entre eux au moins ne présentant pas une totale symétrie par rapport à son axe longitudinal,
- des billes (9, 10, 13), en un matériau non radiotransparent disposées dans ledit noyau (4),
- au moins deux faces latérales amovibles (18, 21) opposées l'une à l'autre renfermant des fils métalliques définissant des figures géométriques.

2. Fantôme selon la revendication 1, **caractérisé en ce qu'**il est de forme générale cubique.

3. Fantôme selon la revendication 1 ou 2, **caractérisé en ce que** l'une (13) des billes est placée au centre du noyau (4).

4. Fantôme selon l'une des revendications 1 à 3, **caractérisé en ce qu'**il comporte six faces latérales (18-23) amovibles renfermant des fils métalliques définissant des figures géométriques.

## Claims

1. Phantom for quality control of a virtual simulation system for a radiotherapy treatment comprising a medical imaging device, **characterised in that** it comprises:
• a support box (1),
• a core (4) arranged in said support box (1) and formed by a plurality of elements (5, 6, 7, 8,11,12,14, 15, 16, 17) of different shapes, dimensions and densities, said densities simulating the densities of various organs and environments of the human body, wherein two of these elements (11, 12) are in the form of two truncated pyramids of different densities inserted one inside the other, and at least one of these does not have complete symmetry in relation to its longitudinal axis,
• balls (9, 10, 13) made of a non-radiotransparent material arranged in said core (4),
• at least two detachable side faces (18,21) located opposite one another to enclose metal wires defining geometric figures.

2. Phantom according to Claim 1, **characterised in that** it has a generally cubic form.

3. Phantom according to Claim 1 or 2, **characterised in that** one (13) of the balls is placed at the centre of the core (4).

4. Phantom according to one of Claims 1 to 3, **characterised in that** it comprises six detachable side faces (18-23) enclosing metal wires defining geometric figures.

## Patentansprüche

1. Phantom für die Qualitätskontrolle eines Systems zur virtuellen Simulation einer radiotherapeutischen Behandlung anhand einer Vorrichtung zur medizinischen Bildgebung, **dadurch gekennzeichnet, dass** sie folgende Teile umfasst:
- einen Stützkasten (1),
- einen Kern (4) im besagten Stützkasten (1) und bestehend aus einer Vielzahl verschiedener Elemente (5, 6, 7, 8, 11, 12, 14, 15, 16, 17) verschiedener Formen, Dimensionen und Dichten, wobei die besagten Dichten die Dichten unterschiedlicher Organe und Milieus des menschlichen Körpers simulieren, wobei zwei dieser Elemente (11, 12) aus zwei ineinandergeschachtelten Pyramidenstümpfen verschiedener Dichten bestehen, wobei mindestens einer von diesen keine Gesamtsymmetrie zu seiner Längsachse aufweist,
- Kugeln (9, 10, 13), die aus einem röntgenstrahlenundurchlässigen Material bestehen und in dem besagten Kern vorgesehen sind,
- mindestens zwei abnehmbaren einander gegenüberliegenden Seitenflächen (18, 21), die geometrische Figuren bestimmende Drähte enthalten.

2. Phantom nach Anspruch 1, durch eine würfelförmige Gesamtform gekennzeichnet.

3. Phantom nach Anspruch 1 oder 2, **dadurch gekennzeichnet, dass** eine (13) der Kugeln im innersten des Kerns angeordnet ist.

4. Phantom nach einem der Ansprüche 1 bis 3, durch sechs abnehmbare Seitenflächen (18-23) gekennzeichnet, die geometrische Figuren bestimmende Drähte enthalten.
